Europäisches Patentamt

**European Patent Office**

Office européen des brevets

(11) Numéro de publication : **0 454 595 A1**

## (12) DEMANDE DE BREVET EUROPEEN

(21) Numéro de dépôt : **91420125.6**

(22) Date de dépôt : **12.04.91**

(51) Int. Cl.⁵ : **C05F 9/04, A01K 67/033, A01G 9/24**

(30) Priorité : **25.04.90 FR 9005744**

(43) Date de publication de la demande :
**30.10.91 Bulletin 91/44**

(84) Etats contractants désignés :
**AT BE CH DE DK ES FR GB GR IT LI LU NL SE**

(71) Demandeur : **SOCIETE DE VALORISATION DES DECHETS SOVADEC**
**Roussas**
**F-26230 Grignan (FR)**

(72) Inventeur : **Chaussinand, Denis**
**12bis Boulevard du Péché**
**F-26200 Montelimar (FR)**
Inventeur : **Lavis, Christian**
**Quartier Bellefontaine**
**F-07220 Viviers (FR)**

(74) Mandataire : **Guerre, Dominique et al**
**Cabinet Germain et Maureau 20 Boulevard Eugène Deruelle BP 3011**
**F-69392 Lyon Cédex 03 (FR)**

(54) **Procédé et installation de traitement de déchets par lombricompostage.**

(57)     Procédé de traitement par lombricompostage de déchets organiques dans au moins une unité de lombricompostage (2), selon lequel dans ladite unité on établit un tas vertical (21) d'un substrat composé d'au moins une couche supérieure (22) de déchets organiques introduits par le haut, d'au moins une couche inférieure (23) de résidus organiques évacués par le bas, et d'une population de lombriciens dispersée dans le tas (21) et progressant vers le haut, la surface externe du tas constituant pour l'essentiel le seul interface d'échange du substrat avec l'extérieur.

    On dispose l'unité (2) de lombricompostage dans une enceinte (1) la séparant de l'air ambiant, et on établit une atmosphère contrôlée (24) dans l'enceinte, directement au contact de la surface latérale du tas (21).

EP 0 454 595 A1

La présente invention concerne le traitement de déchets organiques, domestiques, industriels, agricoles, etc... , par lombricompostage, en vue d'obtenir des résidus organiques, tels que terreau ou humus, ayant une utilité ou valeur, par exemple pour amender un sol.

Le lombricompostage s'entend de toute fermentation aérobie de matières organiques, en présence de lombriciens, plus familièrement vers de terre, contribuant à une pulvérisation, une aération, et un mélange du substrat en cours de fermentation.

Conformément à la demande de brevet français N° 89 14616 déposée au nom de la Demanderesse, on a décrit un procédé de traitement par lombricompostage, selon lequel, dans une unité consistant en un bac à paroi latérale perforée du type grillage, on établit un tas ou charge vertical d'un substrat en fermentation et transformation, composé d'une ou plusieurs couches supérieures de déchets organiques introduits séquentiellement par le haut du bac, et d'une ou plusieurs couches inférieures de déchets organiques évacués séquentiellement par le bas du même bac ; et un population de lombriciens est dispersée dans le tas, en progressant vers le haut, chaque nouvelle évacuation et chaque nouvelle introduction dans le bac étant faite en fonction de la progression des vers vers le haut. Selon ce procédé, en dehors du mouvement vers le bas des différente couches de substrat, ce dernier demeure en masse et n'est agité par aucun moyen mécanique extérieur, et il n'est injecté dans cette masse aucun courant liquide ou gazeux. Ceci veut dire que pour l'essentiel la surface externe, et notamment la surface latérale du tas constituent le seul interface d'échange du substrat avec l'extérieur.

De manière traditionnelle, le lombricompostage est réalisé dans des décharges à ciel ouvert, et dépend pour son efficacité des conditions ambiantes, c'est-à-dire celles de l'atmosphère extérieure, de température et d'humidité, essentiellement. Or ces conditions varient entre le jour et la nuit, et d'une saison à une autre, alors que les vers de terre utilisés pour le lombricompostage ont besoin pour leur vie et développement de conditions stables de température, d'humidité, et d'oxygénation, par exemple entre 25 et 30°C, entre 50 et 70 % d'humidité, et au moins 15 % d'oxygène, pour l'espèce des EISENIA ANDREI.

L'action épuratrice des lombriciens est donc dépendante du milieu ambiant, et toute altération de ce dernier, d'une part entraîne un rendement de transformation faible, et d'autre part conduit au développement de réactions secondaires indésirables, par exemple de fermentation anaérobie conduisant à des gaz malodorants ; et il peut y avoir apparition et développement d'agents pathogènes dans le substrat ou compost.

Partant du procédé décrit par la demande de brevet français N° 89 14616, la présente invention a pour objet des conditions opératoires permettant d'optimiser les conditions de vie et développement des lombriciens.

Selon la présente invention, on dispose une unité de lombricompostage, ou une batterie ou pluralité desdites unités dans une seule et même enceinte la séparant, ou les séparant de l'air ambiant, et on établit une atmosphère contrôlée dans l'enceinte, directement au contact de la surface latérale du tas, ou des tas en fermentation et transformation dans les différentes unités.

Préférentiellement, l'atmosphère contrôlée consiste en un flux d'air contrôlé, circulant d'une entrée à une sortie de l'enceinte, au contact du ou des tas de substrat. Et, on régule au moins l'un des paramètres suivants de contrôle, à savoir teneur en oxygène, teneur en dioxyde de carbone, et température, et on règle le flux d'air circulant dans l'enceinte pour maintenir le ou les paramètres de contrôle à une valeur ou dans une zone préétablie.

L'existence d'une atmosphère contrôlée au contact des tas de substrat permet de piloter par l'extérieur le processus de fermentation ou compostage, la teneur en oxygène ou eau par exemple de l'atmosphère contrôlée déterminant les taux de transfert de ces éléments vers le substrat, et par conséquent les vers de terre.

La présente invention comporte également les modes d'exécution suivants.

Le flux d'air introduit dans l'enceinte est prélevé sur une fosse de réception des déchets organiques à lombricomposter. Ceci permet d'éviter la libération de gaz malodorants dans l'atmosphère, en les désodorisant lors de leur passage à l'intérieur de l'enceinte, où ils sont oxydés par voie chimique et/ou biochimique.

Par ailleurs, on régule les paramètres de contrôle, en fonction des conditions climatiques à l'extérieur de l'enceinte. Ceci permet en particulier de tenir compte des cycles nycthémériaux thermiques, pour permettre par exemple un renouvellement de l'air introduit dans l'enceinte, aux périodes les plus fraîches ou les plus chaudes, selon les conditions climatiques.

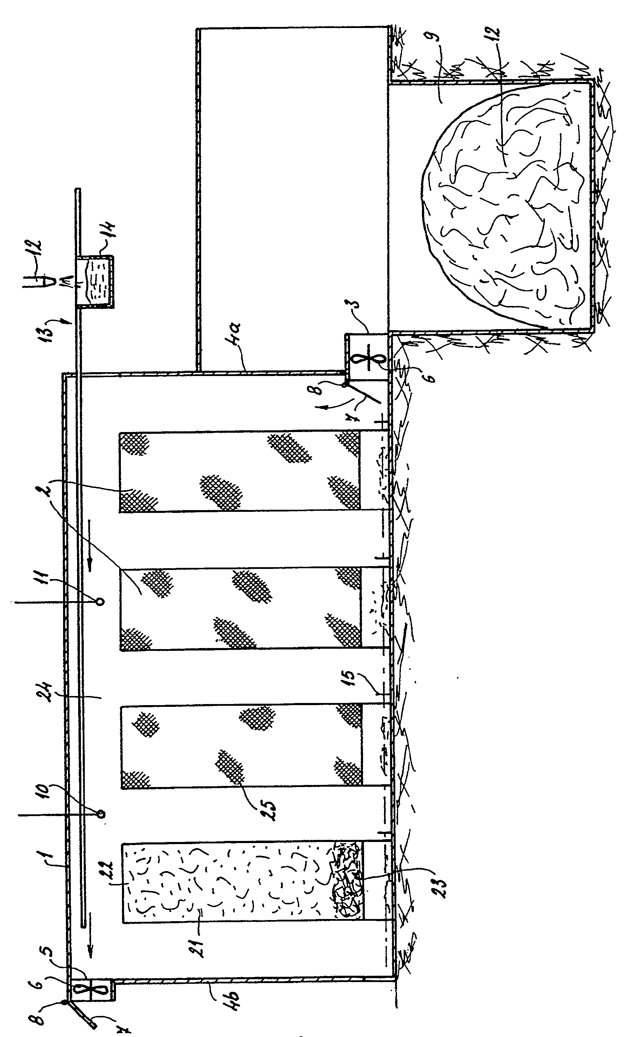

De toutes façons, l'invention sera bien comprise à l'aide de l'unique figure représentant l'ensemble de l'installation pour la mise en oeuvre du procédé.

Comme illustré au dessin, cette installation comprend une enceinte 1 à l'intérieur de laquelle se trouvent quatre unités de lombricompostage 2. Chaque unité de lombricompostage 2 comprend un bac 25 dont la paroi latérale, grillage par exemple, est perméable aux gaz et aux liquides; dans chaque bac est disposée une charge 21, pile ou tas vertical d'un substrat, composé de couches supérieures 22 de déchets organiques introduits par le haut, et de couches inférieures 23 de résidus organiques évacués par le bas. Chaque unité de lombricompostage 2 contient à la fois la matière organique à composter, mais aussi la population des lombriciens chargée d'en effectuer la transformation, et se déplaçant progressivement vers le haut. Il s'agit de lombriciens qui appartiennent par exemple à l'espèce des EISENIA ANDREI, et qui vivent et se développent dans des conditions de températures comprises entre 25 et 28°C, une humidité relative de 50 à 70%, et un taux d'oxygènation de l'air d'au moins 15%.

L'introduction des déchets organiques à composter, qui ont été préalablement humidifiés par de l'eau sous la buse 12, par le haut des bacs ou unités de lombricompostage 2, se fait de manière contrôlée et discontinue, par l'intermédiaire d'un pont roulant 13 comportant des godets 14.

Bien évidemment, il est tout à fait envisageable d'utiliser d'autres moyens d'introduction, tels que par exemple un tapis roulant.

L'évacuation des résidus organiques du lombricompostage s'effectue par le bas de chaque unité de lombricompostage 2, par une chaîne comportant des raclettes 15. Ces résidus lombricompostés sont ainsi évacués à l'extérieur de l'enceinte 1, pour être convoyés par un transporteur à bande, non représenté, dans un récipient de réception, avant leurs traitements ultérieurs.

Pour l'agencement et le fonctionnement des unités de lombricompostage, on se référera plus particulièrement à la demande de brevet français N° 89 14616, dont le contenu est incorporé à la présente demande, en tant que de besoin.

Cette enceinte 1 comprend un orifice 3 ménagé dans la partie inférieure de l'une de ses parois latérales 4a, et un orifice 5 ménagé dans la partie supérieure d'une autre de ses parois latérales 4b. Chaque orifice 3,5 comporte un ventilateur à pales 6, et il est plus ou moins obturable par un volet 7 mobile autour d'une charnière 8. Ces moyens permettent de faire circuler un flux d'air dans l'enceinte 1, établissant une atmosphère contrôlée dans cette dernière.

Pour réguler l'un au moins des paramètres suivants dans l'enceinte 1, à savoir teneur en oxygène, teneur en dioxyde de carbone, température et humidité, de l'air frais, provenant notamment d'une fosse de réception de déchets 9 à lombricomposter, est introduit à l'intérieur de cette enceinte 1 par le ventilateur 6, disposé notamment au-dessus de la fosse 9. Cet air frais, et notamment les molécules malodorantes qu'il contient, est utilisé comme source de chaleur, d'énergie et éléments biogènes, après leur dégradation par des micro-organismes contenus dans les matières organiques à composter, par les lombriciens.

La dégradation des molécules organiques complexes des déchets, en molécules simples, par fermentation et oxydation est exothermique. Elle entraine une augmentation de la température de l'air à l'intérieur de l'enceinte. De plus, elle dégage du dioxyde de carbone et de l'eau, ce qui conduit à une augmentation de la teneur relative de ceux-ci dans l'atmosphère à l'intérieur de l'enceinte. Cet air chaud, chargé en dioxyde de carbone et vapeur d'eau, est alors évacué à l'extérieur de l'enceinte par le ventilateur 6 disposé dans l'orifice 5.

Cet air chaud évacué peut être utilisé comme fluide énergétique et comme agent de croissance pour permettre un développement accéléré de végétaux dans une serre non représentée. En effet, la fonction chlorophylienne qui conditionne la croissance des végétaux est favorisée par un air contenant une teneur relative en dioxyde de carbone élevée, par exemple supérieure à 0,3%, une humidité relative importante comprise entre 90 % et 100 % et une température supérieure à 30°C.

Pour contrôler la teneur en dioxyde de carbone et en oxygène de l'atmosphère gazeuse à l'intérieur de l'enceinte 1, ainsi que le taux d'humidité relative et la température de celui-ci, des capteurs 10, 11 du type capteur inductif, capacitif ou autre, sont disposés à l'intérieur de cette enceinte. Ils transmettent des signaux représentatifs des paramètres de contrôle retenus à un moyen de calcul et de programmation tel qu'un ordinateur ou un automate programmable non représenté. Ce moyen de calcul, en fonction des informations reçues des capteurs, va déclencher une ouverture plus ou moins importante d'au moins un volet 7, ou un fonctionnement d'au moins un ventilateur 6, ce qui va permettre l'amenée d' un flux d'air plus ou moins important à l'intérieur de l'enceinte, rétablissant le ou les paramètres de contrôle à une valeur ou dans une zone préétablie. De cette manière, on peut maitenir l'atmosphère dans l'enceinte 1 à un seuil minimum d'oxygénation, caractérisé par une valeur minimum du taux d'oxygène ou une valeur maximum du taux de dioxyde de carbone. On peut aussi maintenir l'atmosphère à un seuil minimum de température.

Bien évidemment, cet automate peut, grâce aux programmes qu'il contient, réguler les paramètres de contrôle à l'intérieur de l'enceinte, en fonction des conditions climatiques extérieures, qui lui sont fournies par un capteur de température non représenté. De ce fait, les paramètres de contrôle peuvent être régulés, en tenant compte des cycles nycthémériaux thermiques par exemple, pour permettre un renouvellement de l'air aux périodes les plus fraiches, en fonction des conditions climatiques.

De même, les cycles de renouvellement d'air peuvent tenir compte des heures de déchargement des déchets à l'intérieur de la fosse 9.

Le contrôle de la teneur en dioxyde de carbone, à l'intérieur de l'enceinte 1, permet aussi d'indiquer si le franchissement d'un seuil critique est atteint, et ainsi d'interdire tout accès à l'intérieur de cette enceinte au personnel, en raison de la nocivité pour l'organisme de ce gaz.

Le contrôle de l'humidité à l'intérieur de l'enceinte 1 peut s'effectuer en réglant l'aspersion 12 des déchets, ou le déplacement de la bande 13.

Pour terminer, la circulation du flux d'air peut s'effectuer par simple thermosiphon.

## Revendications

1. Procédé de traitement par lombricompostage de déchets organiques dans au moins une unité de lombricompostage (2), selon lequel dans ladite unité on établit un tas vertical (21) d'un substrat composé d'au moins une couche supérieure (22) de déchets organiques introduits par le haut, d'au moins une couche inférieure (23) de résidus organiques évacués par le bas, et d'une population de lombriciens dispersée dans le tas (21) et progressant vers le haut, la surface externe du tas constituant pour l'essentiel le seul interface d'échange du substrat avec l'extérieur, caractérisé en ce que on dispose l'unité (2) de lombricompostage dans une enceinte (1) la séparant de l'air ambiant, et on établit une atmosphère contrôlée (24) dans l'enceinte, directement au contact de la surface latérale du tas (21).

2. Procédé selon la revendication 1, caractérisé en ce que l'atmosphère contrôlée (24) consiste en un flux d'air contrôlé, circulant d'une entrée (3) à une sortie (5) de l'enceinte, au contact du tas (21) de substrat.

3. Procédé selon la revendication 2, caractérisé en ce qu'on régule au moins l'un des paramètres suivants de contrôle, à savoir teneur en oxygène, teneur en dioxyde de carbone, humidité, et température, et on règle le flux d'air circulant dans l'enceinte pour maintenir le paramètre de contrôle à une valeur ou dans une zone préétablie.

4. Procédé selon la revendication 2, caractérisé en ce qu'on règle le flux d'air par le débit introduit et/ou le débit extrait de l'enceinte.

5. Procédé selon la revendicaiton 2, caractérisé en ce que le flux d'air introduit dans l'enceinte est prélevé sur une fosse (9) de réception des déchets organiques à lombricomposter.

6. Procédé selon la revendication 1, caractérisé en ce qu'on contrôle l'humidité du tas, par trempage (12) ou aspersion des déchets avant leur introduction sur le tas.

7. Procédé selon la revendication 2, caractérisé en ce qu'on régule le paramètre de contrôle, en fonction des conditions climatiques à l'extérieur de l'enceinte.

8. Procédé selon la revendication 2, caractérisé en ce qu'on contrôle l'atmosphère dans l'enceinte à un seuil minimum d'oxygénation, caractérisé par une valeur minimum du taux d'oxygène ou une valeur maximum du taux de dioxyde de carbone de l'atmosphère contrôlée.

9. Procédé selon la revendication 2, caractérisé en ce qu'on contrôle l'atmosphère dans l'enceinte à un seuil maximum de température.

10. Installation pour la mise en oeuvre du procédé selon la revendication 1, comprenant une unité (2) de lombricompostage, comprenant un bac (25) dont la paroi latérale au moins est perméable, des moyens (13) pour introduire des déchets organiques par le haut, dans le bac, et des moyens (15) pour évacuer des résidus organiques par le bas, du même bac, caractérisé en ce qu'une pluralité d'unités (2) de lombricompostage est disposée dans une même enceinte (1) les séparant de l'air ambiant, et permettant d'établir une atmosphère contrôlée directement au contact des différents tas (21) contenus par les différents bacs.

EP 0 454 595 A1

Office européen
des brevets

**RAPPORT DE RECHERCHE EUROPEENNE**

Numero de la demande

EP    91 42 0125

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-3644710 (F. HOURANI) <br> * colonne 3, ligne 50 - colonne 4, ligne 36; revendications * | 1, 10 | C05F9/04 <br> A01K67/033 <br> A01G9/24 |
| A | GB-A-1065571 (DEUTSCHE BABCOCK & WILCOX-DAMPFKESSELWERKE AKTIENGESELLSCHAFT) <br> * revendications * | 1, 10 | |
| A | US-A-3010801 (K.L. SCHULZE) <br> * revendications * | 1, 10 | |
| A | US-A-3820278 (J.A. GIASANTE) <br> * colonne 8, ligne 55 - colonne 9, ligne 10; revendications * | 1, 10 | |
| A | EP-A-129520 (CONSIGLIO NAZIONALE DELLE RICERCHE) <br> * revendications * | 1, 10 | |
| A | Compendium on solid waste management by vermicomposting, August 1980, Contract Number 68-03-2803, Camp Dresser & McKee, Inc., Boston, Massachusetts, USA <br> * p. 12-14 * | 1-10 | **DOMAINES TECHNIQUES RECHERCHES (Int. Cl.5)** |
| A | EP-A-91495 (R. SOLI) <br> * page 5, lignes 6 - 10; revendications ; figures 1, 4 * | 10 | C05F <br> A01K |
| A | AT-B-390053 (J. PISCHINGER) | | |

Le présent rapport a été établi pour toutes les revendications

| Lieu de la recherche | Date d'achèvement de la recherche | Examinateur |
|---|---|---|
| LA HAYE | 05 JUILLET 1991 | SCHUT R.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)